# DEMANDE DE BREVET EUROPEEN

(11) **EP 4 474 003 A1**
(43) Date de publication de la demande: **11.12.2024**
(21) Numéro de dépôt: 24180040.8
(22) Date de dépôt: 04.06.2024
(51) Int. Cl.: A61N 5/06

(54) **DISPOSITIF DE TRAITEMENT PAR PHOTOTHÉRAPIE**

(30) Priorité: 07.06.2023 FR 2305755
(71) Demandeur: Lucibel SA, 76360 Barentin (FR)
(72) Inventeur: GROS-DAILLON, Thibault, 76590 Criquetot-sur-Longueville (FR); MOA, Yacine, 76510 Saint Nicolas d'Aliermont (FR)
(74) Mandataire: Martin, Marie-Aude

(57) **Abrégé**

Le support pour un casque (20) de traitement photothérapie incorporant une source de lumière émettant à l'intérieur du casque (20) pour traiter le cuir chevelu de la tête d'un utilisateur. Le support (40) comprend une base (44) pour alimenter le casque (20) pendant le traitement, la base (44) comprenant une interface utilisateur (440) configurée pour contrôler la source de lumière du casque (20), et comprend un bras (42) s'étendant selon une direction sensiblement verticale monté à une première extrémité sur la base (44) et prolongé à une deuxième extrémité par un siège de support du casque (20) s'étendant selon une direction sensiblement horizontale.

## Description

La présente invention concerne un dispositif de traitement par photothérapie pour une région externe du corps d'un être vivant. Plus particulièrement mais non exclusivement, elle s'applique au traitement par photothérapie de la surface de tête d'un utilisateur, par exemple au traitement du cuir chevelu en tant que traitement de la calvitie.

De façon connue en soi, le traitement consiste en l'application d'un rayonnement lumineux présentant un spectre d'émission prédéfini directement sur le cuir chevelu. Ce traitement par photothérapie peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Le rayonnement est de préférence sensiblement monochromatique ou présente un spectre d'émission compris dans une bande relativement étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau et le traitement du cuir chevelu.

Il a été ainsi observé au cours de nombreuses études scientifiques, qu'un traitement par photothérapie dans une plage de longueurs d'onde prédéfinie, notamment dans la lumière rouge ou bleue, permet d'agir de façon bénéfique sur de nombreux problèmes cutanés, tels que les cicatrices, les vergetures, l'acné, les rides et ridules et de nombreux problèmes capillaires dont l'alopécie.

Plus particulièrement, lors de l'application du rayonnement sur le cuir chevelu, notamment dans la lumière rouge et plus particulièrement autour de la longueur d'onde 630 nm, les cellules de la région irradiées vont réagir selon un principe similaire à celui de la photosynthèse des végétaux. La lumière va stimuler les cellules, et plus spécifiquement certaines structures cellulaires de la cellule, telles que les mitochondries.

De façon connue en soi, les mitochondries jouent un rôle particulièrement important dans le processus de respiration cellulaire ainsi que dans le processus de transformation des nutriments en un vecteur énergétique désigné couramment par ATP (acronyme pour Adénosine Tri Phosphate). Dès lors, la stimulation des mitochondries par la lumière rouge contribue à une régénération des cellules grâce à l'amélioration du métabolisme cellulaire. Une telle régénération cellulaire va se manifester par exemple pour les cellules de la peau du visage par une augmentation de la production de collagène et d'élastine et par conséquent une diminution observable des rides et ridules et pour le cuir chevelu par une stimulation de la repousse capillaire, une amélioration de la qualité et de la densité des cheveux ainsi qu'une stabilisation de la perte de cheveux.

On connaît déjà dans l'état de la technique, en particulier de la demande de brevet EP2477697 un équipement de traitement par photothérapie du cuir chevelu d'un utilisateur, comprenant un casque délimitant une extrémité ouverte par laquelle, en position d'utilisation, la tête de l'utilisateur est au moins partiellement engagée.

Le casque décrit dans ce document présente certains inconvénients significatifs notamment en matière de praticité et d'ergonomie.

Or, les objets de photothérapie sont désormais considérés comme des produits de bien-être et doivent s'intégrer au mieux tant dans le quotidien que dans l'environnement des utilisateurs. Ces objets doivent donc être à la fois ergonomiques mais également esthétiques en offrant un aspect visuel global de qualité premium. Différentes caractéristiques permettent d'apporter une perception de qualité telles que le choix des matériaux, le poids et l'ergonomie.

L'invention propose d'apporter une solution pour améliorer l'utilisation quotidienne de ces objets tout en apportant une perception de qualité, d'ergonomie et de technicité accrue.

### Description de l'invention

A cet effet l'invention a pour objet un support pour un casque de traitement photothérapie incorporant une source de lumière émettant à l'intérieur du casque pour traiter le cuir chevelu de la tête d'un utilisateur, caractérisé en ce qu'il comprend une base pour alimenter le casque pendant le traitement, la base comprenant une interface utilisateur configurée pour contrôler la source de lumière du casque, et comprend un bras s'étendant selon une direction sensiblement verticale monté à une première extrémité sur la base et prolongé à une deuxième extrémité par un siège de support du casque s'étendant selon une direction sensiblement horizontale.

Dans un mode de réalisation préféré de l'invention, le siège ayant une forme générale oblongue définissant des directions longitudinale et transversale croisées, le casque est configuré pour être porté par le support de telle sorte qu'en configuration de support, la direction longitudinale du support s'étende selon un axe s'étendant transversalement entre deux zones latérales auriculaires du casque.

Dans un mode de réalisation préféré de l'invention, le siège a une forme générale courbe avec des bords tombants selon la direction longitudinale et est configuré pour soutenir le casque dans une partie sommitale.

Dans un mode de réalisation préféré de l'invention, le siège se présente sous une forme de plaque ovoïde recourbé arrondie sur les bords périphériques.

Dans un mode de réalisation préféré de l'invention, la surface de contact avec le casque du siège de support pour former une surface d'adhérence du casque sur le support.

Dans un mode de réalisation préféré de l'invention, le siège comprend une couche de silicone surmoulée sur une surface d'adhérence avec le casque.

Dans un mode de réalisation préféré de l'invention, la surface du siège en contact avec le casque comprend une pluralité pions anti-dérapants sur la surface supérieure du support.

Dans un mode de réalisation préféré de l'invention, la base est un socle lesté par exemple par une pièce de platine logée à l'intérieur d'un boîtier.

Dans un mode de réalisation préféré de l'invention, la base enferme une carte électronique de contrôle de la source lumineuse du casque selon un protocole prédéfini.

L'invention a encore pour objet un dispositif de traitement par photothérapie d'une surface de cuir chevelu d'un utilisateur, comprenant un casque et un support du casque, caractérisé en ce que le support est selon l'invention.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La [Fig.1] est une vue en perspective d'un ensemble comprenant un casque et un support de casque selon l'invention ;
[Fig.2] La [Fig.2] est une vue en perspective du casque de la [Fig.1] ;
[Fig.3] La [Fig.3] est une vue de dessous du casque de la [Fig.2] ;
[Fig.4] La [Fig.4] est une vue en perspective du support de la [Fig.1] ;
[Fig.5] La [Fig.5] est une vue de face du support de la [Fig.1] ;
[Fig.6] La [Fig.6] est une vue de côté du support de la [Fig.1] ;
[Fig.7] La [Fig.7] est une vue en perspective du support de la [Fig.1] dans lequel un couvercle d'un socle du support est séparé d'un fond du socle ;
[Fig.8] La [Fig.8] est une vue à échelle agrandie de l'intérieur du socle du support.

### Description détaillée de l'invention

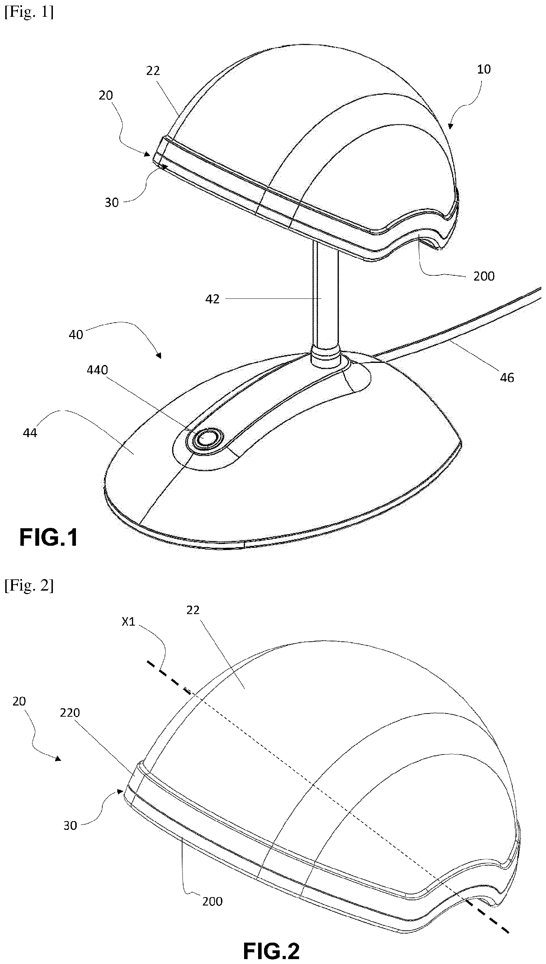

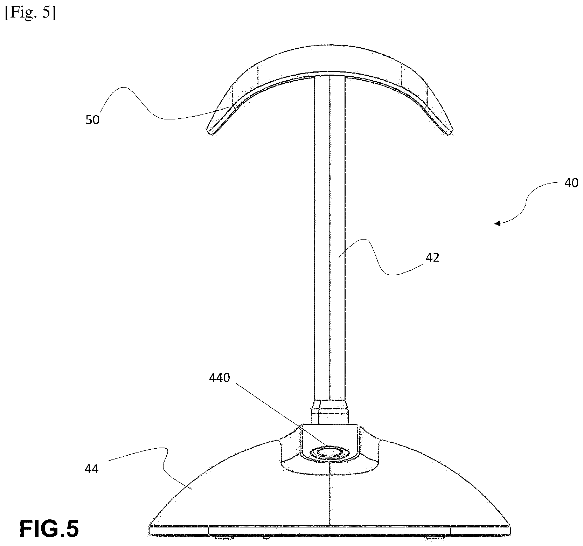

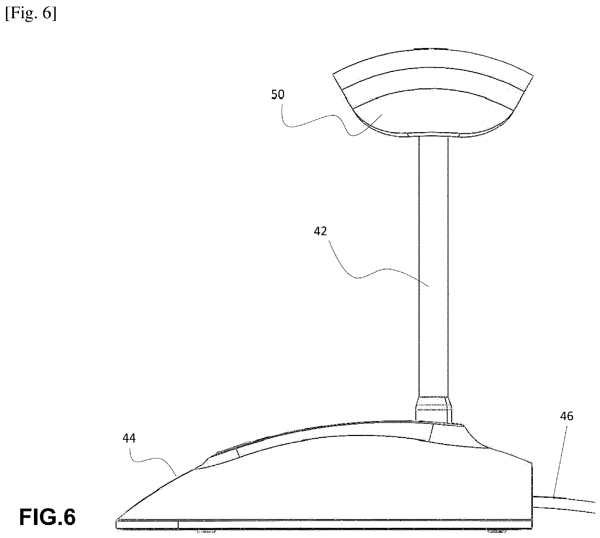

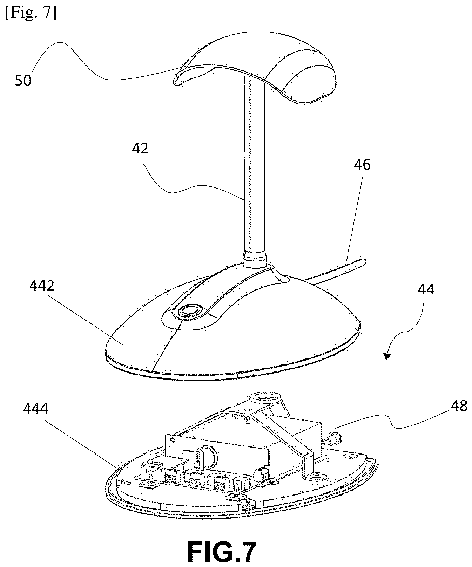

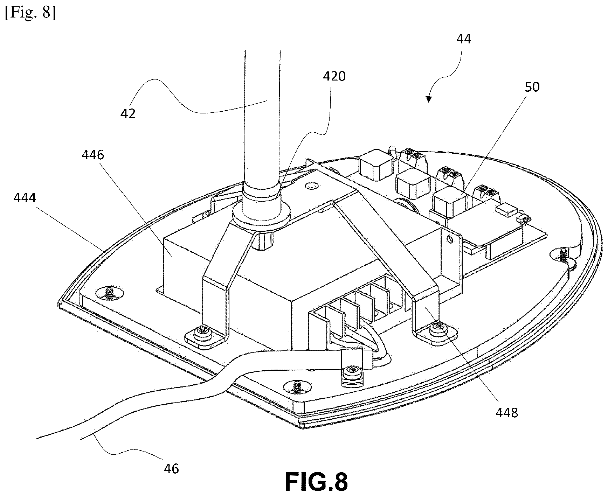

On a représenté sur les figures 1 à 8 un dispositif de photothérapie selon l'invention. Le dispositif est désigné par la référence générale 10.

Comme cela est représenté sur la [Fig.1], le dispositif 10 comprend un casque de photothérapie 20 et un support 40 du casque 20 portant la référence 14.

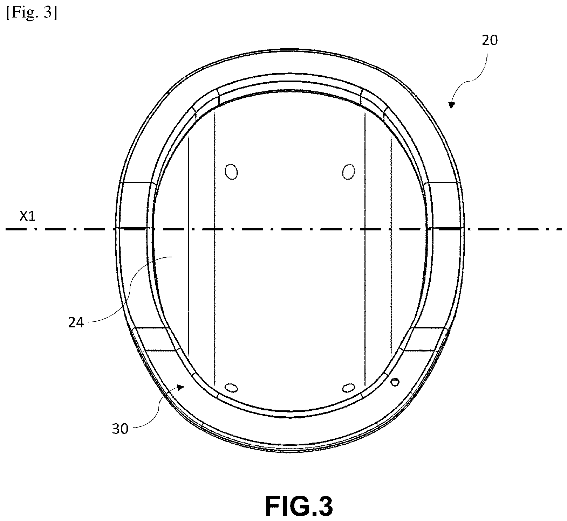

Le casque 20 est illustré en détail en [Fig.2] et en [Fig.3]. Dans l'exemple illustré, le casque 20 comprend un corps 22 de forme générale hémisphérique délimitant une ouverture pour le passage de la tête d'un utilisateur. Le corps 22 délimite également une cavité intérieure qui permet de couvrir une surface du cuir chevelu de l'utilisateur. Le corps 22 du casque 20 incorpore de façon classique une source de lumière (non visible sur les figures) qui émet en direction de la surface à traiter vers l'intérieur du casque 20. La source de lumière comprend dans cet exemple une pluralité de diodes électroluminescentes émettant de préférence une lumière rouge avec une longueur d'onde autour de 630 nanomètres, comprise par exemple dans une plage de 620 à 640 nanomètres. En variante, d'autres longueurs d'onde de lumière peuvent être utilisées en fonction de la nature du traitement de photothérapie souhaité.

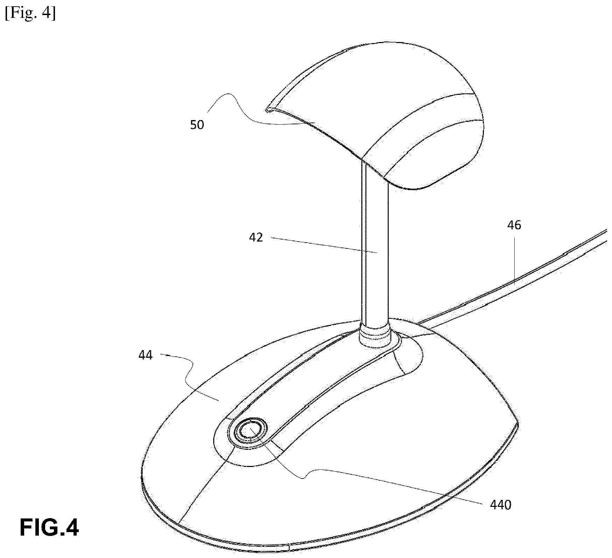

On a représenté en détail sur la [Fig.4] le support 40 selon un mode de réalisation préféré de l'invention. Le support 40 comprend une base 44 pour alimenter électriquement la source de lumière du casque 20 pendant le traitement. A cet effet, la base 44 enferme par exemple une carte électronique de contrôle 448 de la source lumineuse du casque 20 selon un protocole prédéfini.

Comme cela est illustré sur la [Fig.4], le support 40 comprend également un câble d'alimentation 46 de la carte électronique 448 de la base 44 avec une source d'alimentation extérieure, par exemple l'alimentation générale d'un réseau électrique domestique.

Par ailleurs, la base 44 comprend des moyens 48 de connexion du casque 20 à la base 44. Ces moyens de connexion 48 comprennent par exemple une prise de connexion électrique, telle qu'une prise USB, une prise Jack, etc., à laquelle est branché un câble d'alimentation (non visible sur les figures) du casque 20.

La base 44 comprend en outre une interface utilisateur 440. Dans l'exemple illustré sur les figures, l'interface utilisateur 440 est supportée matériellement directement sur la base 44 du dispositif 10 par exemple par un bouton tactile ou non ou encore en variante par exemple par des curseurs et molettes de réglage des boutons implémentés matériellement sur le socle 44. Cette interface utilisateur 440 permet de commander l'allumage et l'extinction de la source de lumière du casque 10.

Bien entendu, d'autres interfaces utilisateur peuvent être implémentées sans sortir du cadre de l'invention, par exemple un écran tactile ou une application logicielle implémentée sur un équipement extérieur. Ainsi, dans une variante non illustrée, le socle du support comprend une fenêtre d'affichage formant l'interface utilisateur. Dans une autre variante non illustrée sur les figures, l'interface utilisateur est supportée de manière logicielle sur une tablette (non illustrée). La tablette communique par exemple avec le dispositif 10 par une liaison filaire ou sans fil (par exemple par Bluetooth^{®} ou par WiFi), via un module de contrôle logé dans le socle.

En particulier, le support 40 comprend un bras 42, tel qu'une tige autoportante 42, monté à une première extrémité 422 sur la base 44 et prolongé à une deuxième extrémité 424 par un siège 50 de support du casque 20 dans une position d'équilibre. Ce bras 42 s'étend sensiblement verticalement dans une configuration d'utilisation du support. Le siège 50 s'étend dans une direction sensiblement horizontale.

La base 44 comprend un boîtier formant socle, comme cela est illustré sur la [Fig.3] sur laquelle la tige 42 est fixée par son extrémité 420. Par exemple, la base 44 comprend un étrier 448 de fixation de la tige 42. Le boîtier comprend un couvercle 442 monté sur un fond 444.

La base 44 est de préférence lestée par exemple par une pièce de platine 446 logée à l'intérieur du boîtier formant le socle du support 40. Ainsi, la base 44 a à la fois une fonction de contrepoids pour former un support stable pour le casque 20 et une fonction de base de commande de la source de lumière.

De préférence, comme cela est visible sur la [Fig.3], le siège 50 a une forme générale oblongue et définit une direction longitudinale 52 et une direction transversale 54 croisées. De préférence, le casque 20 est configuré pour être porté par le support 50 de telle sorte qu'en configuration de support, la direction longitudinale du support 50 s'étende selon un axe X1 transversal s'étendant entre les deux zones latérales auriculaires 210 du casque 20. Par exemple, chaque zone latérale auriculaire 210 délimite une excursion latérale en forme de lobe pour le contournement de l'oreille.

De préférence, le siège 50 est pourvu d'une conformation complémentaire de forme lorsque le casque 20 est associé au support 40. Le siège 50 est configuré pour se positionner en regard de la position sommitale du casque 20. Ainsi, cette conformation permet de suggérer de manière naturelle et intuitive le positionnement du casque 20 sur le support 40.

Comme cela est illustré de façon non limitative sur les figures, le siège 50 a une forme générale courbe, par exemple en forme de calotte convexe configuré pour soutenir le casque 20 dans sa partie sommitale.

La calotte se présente sous une forme d'une plaque ovoïde recourbée arrondie sur les bords périphériques, et notamment sur ses deux bords d'extrémité transversaux. Cette forme de languette arrondie sur ses deux bords latéraux transversaux permet d'épouser la courbure interne du casque 20.

Afin d'éviter une déstabilisation du casque 20, de préférence, la surface de contact avec le casque 20 du siège 50 de support du casque 20 a des propriétés d'adhérence du casque 20 sur le support 40. Par exemple, le siège 50 comprend une couche de silicone surmoulée pour former une surface d'adhérence avec le casque 20. En variante, la surface du siège 50 comprend une pluralité pions anti-dérapants sur la surface supérieure du support 40.

On va maintenant décrire les principaux aspects de fonctionnement du dispositif de photothérapie selon l'invention.

Le casque 10 est posé en équilibre sur le support 40, dans le cas présent sur le siège 50 prévu à cet effet. Ce siège 50 présente des directions croisées qui sont telles que le casque est parfaitement soutenu selon l'axe X1 et que le casque 10 pivote légèrement vers sa position finale par basculement sous l'effet de la gravité autour de cet axe X1 afin de venir naturellement dans une position d'équilibre stable.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Support (40) pour un casque (20) de traitement photothérapie incorporant une source de lumière émettant à l'intérieur du casque (20) pour traiter le cuir chevelu de la tête d'un utilisateur, **caractérisé en ce qu'**il comprend une base (44) pour alimenter le casque (20) pendant le traitement, la base (44) comprenant une interface utilisateur (440) configurée pour contrôler la source de lumière du casque (20), et comprend un bras (42) s'étendant selon une direction sensiblement verticale monté à une première extrémité sur la base (44) et prolongé à une deuxième extrémité par un siège (50) de support du casque (20) s'étendant selon une direction sensiblement horizontale.

2. Support (40) selon la revendication précédente, dans lequel le siège (50) ayant une forme générale oblongue définissant des directions longitudinale et transversale croisées, le casque (20) est configuré pour être porté par le support (40) de telle sorte qu'en configuration de support, la direction longitudinale du support (40) s'étende selon un axe (X1) s'étendant transversalement entre deux zones latérales auriculaires du casque (20).

3. Support (40) selon l'une quelconque des revendications précédentes, dans lequel le siège (50) a une forme générale courbe avec des bords tombants selon la direction longitudinale et est configuré pour soutenir le casque (10) dans une partie sommitale.

4. Support (40) selon l'une quelconque des revendications précédentes, dans lequel le siège (50) se présente sous une forme de plaque ovoïde recourbé arrondie sur les bords périphériques.

5. Support (40) selon la revendication précédente, dans lequel la surface de contact avec le casque (10) du siège (50) de support pour former une surface d'adhérence du casque (10) sur le support.

6. Support (40) selon la revendication précédente, dans lequel le siège (50) comprend une couche de silicone surmoulée sur une surface d'adhérence avec le casque.

7. Support (40) selon la revendication 5 ou 6, dans lequel la surface du siège (50) en contact avec le casque (10) comprend une pluralité pions anti-dérapants sur la surface supérieure du support (40).

8. Support (40) selon l'une quelconque des revendications précédentes, dans lequel la base (44) est un socle lesté par exemple par une pièce de platine (446) logée à l'intérieur d'un boîtier (442, 444).

9. Support (40) selon l'une quelconque des revendications précédentes, dans lequel la base (44) enferme une carte électronique de contrôle de la source lumineuse du casque (10) selon un protocole prédéfini.

10. Dispositif (10) de traitement par photothérapie d'une surface de cuir chevelu d'un utilisateur, comprenant un casque (20) et un support du casque (40), **caractérisé en ce que** le support (40) est selon l'une quelconque des revendications 1 à 9.
